# EUROPEAN PATENT APPLICATION

(11) **EP 2 772 256 A1**
(43) Date of publication of application: **03.09.2014**
(21) Application number: 14168177.5
(22) Date of filing: 05.11.2008
(51) Int. Cl.: A61K 31/473, A61K 31/4045, A61K 31/428, A61K 31/519, A61K 31/551, A61K 31/554, A61P 3/04, A61P 3/10, A61K 45/06, A61K 31/5513

(54) **Compositions for retarding weight gain associated with use of atypical antipsychotic drugs**

(30) Priority: 05.11.2007 US 985563 P
(62) Divisional of application: 08847599.1
(71) Applicant: McIntosh, Diane, Vancouver, British Columbia V6N 2X6 (CA); Kjernisted, Kevin, North Vancouver, British Columbia V7J 2J8 (CA)
(72) Inventor: McIntosh, Diane, Vancouver, British Columbia V6N 2X6 (CA); Kjernisted, Kevin, North Vancouver, British Columbia V7J 2J8 (CA)
(74) Representative: Isarpatent

(57) **Abstract**

Methods and compositions for preventing or reducing weight gain and associated metabolic syndrome in patients receiving atypical antipsychotic drugs for treatment of mental illnesses are described. The invention comprises administering to a patient in need of treatment an effective amount of a dopamine agonist in conjunction with an effective amount of an atypical antipsychotic drug. In one embodiment of the invention, the dopamine agonist is pramipexole. The dopamine agonist may be administered in a low dose, such as less than 1 mg per day of pramipexole. Examples of atypical antipsychotic drugs which may be administered in conjunction with the dopamine agonist include clozapine, olanzapine, quetiapine and risperadone.

## Description

### Reference to Related Applications

This application claims the benefit of the filing date of United States provisional patent application No. 60/985,563 filed 5 November 2007, the entirety of which is hereby incorporated by reference.

### Technical Field

This invention relates to methods and compositions for preventing or reducing weight gain and associated metabolic syndrome in patients receiving atypical antipsychotic drugs for treatment of mental illnesses.

### Background

There are many physical health risks associated with serious mental illnesses and their treatments, aside from the inherent morbidity of illness symptoms and functional decline. Chief amongst those physical health risks is the increased prevalence of obesity and associated metabolic syndrome.[1]

Metabolic syndrome was defined by the World Health Organization (WHO) in 1998 as the presence of type II diabetes or impaired glucose tolerance or insulin resistance associated with two or more of the following: (i) hypertension (>160/90), (ii) elevated triglycerides or low HDL- cholesterol, (iii) body mass index (BMI) > 30kg/m² or increased hip/waist ratio of >0.9 in men or >0.85 in women, and (iv) microalbuminuria of ≥ 20 µg/min overnight. Forty per cent of those with impaired glucose tolerance and 70% of those with type II diabetes have features of metabolic syndrome, and therefore are at risk for the associated three-fold increase in cardiac disease, including myocardial infarction and stroke. While heredity is the greatest predictor of the development of metabolic syndrome, certain mental illnesses *(e.g.* bipolar disorder) and psychotropic medications are also associated with an increased risk. [1,2,3,4]

There are six commonly prescribed atypical antipsychotics available on the North American market. Four (clozapine, olanzapine, quetiapine and risperidone) are associated with significant weight gain, although the amount of weight gain is highly variable, depending on the drug and the individual.[5] In addition to the health risks posed by weight gain and metabolic syndrome, patients may choose to discontinue treatment, often resulting in a worsening of their mental illnesses.

Most antipsychotic drugs function by regulating the activity of neurotransmitters or their receptors. Typical or conventional antipsychotic agents (e.g. haloperidol, chlorpromazine) are dopamine (DA) antagonists. Blockade of the DA2 receptor (D2R) in mesolimbic regions is likely responsible for the diminution of the positive symptoms associated with schizophrenia. However, in mesocortical regions, dopamine blockade results in a potential worsening of negative symptoms and cognitive impairment. In tubulinfundibular areas, dopamine blockade results in increased prolactin and in the nigrostriatum leads to extrapyramidal side effects (EPSEs). Most atypical antipsychotics antagonize 5HT2 receptors to a greater extent than they antagonize D2Rs. This 5HT2 receptor inhibition results in an increase of dopamine release, which in the prefrontal cortex improves negative symptoms and cognition and in the nigrostriatal pathway potentially reduces or eliminates the risk of EPSEs. [6]

Two novel antipsychotics, aripiprazole and ziprazidone, are not commonly associated with weight gain, diabetes or metabolic syndrome.[5] The reason for this difference may lie in the manner in which these drugs affect dopamine since weight loss is associated with dopamine agonism. A reduction of dopamine neurotransmission in the suprachiasmatic nuclei precedes the development of obesity and insulin resistance. Drugs that block D2R and D4R, in animal models, increase appetite and cause weight gain.[7,8,9]

Aripiprazole is a partial dopamine agonist, with high affinity for the D2R. In brain regions where there is an abundance of dopamine available for the receptor, aripiprazole acts as a dopamine antagonist. As a result, in the mesolimbic system, where there is excessive dopamine activity associated with the positive symptoms of schizophrenia, aripiprazole inhibits dopamine. Alternately, aripiprazole acts as a dopamine agonist in mesocortical areas, where in schizophrenia there is a relative dopamine deficit. This results in improvement of negative and cognitive symptoms. Aripiprazole also acts as a high-affinity partial agonist at the 5HT1A receptor (responsible for anxiolysis and antidepressant effects) and an antagonist at the 5HT2 receptor, but not to the same degree as its D2R affinity. 5HT2 antagonism increases dopamine release in the prefrontal cortex and substantia nigra, also improving negative symptoms as well as cognition and extrapyramidal side effects. 5HT2 antagonism also leads to the increase of noradrenolin in the prefrontal cortex. Aripiprazole does not have anticholinergic effects and displays very low antihistaminergic and α1 affinity, which might also explain the low incidence of cognitive dysfunction and orthostatic hypotension associated with this agent.[6]

The reasons why ziprazidone does not cause weight gain have not yet been fully characterized, but may also possibly be related to dopamine effects.

It is well known in the literature that dopamine regulation is associated with weight gain. Dopamine regulates energy balance primarily by modulating food intake via the mesolimbic (reward) and mesohypothalamic (satiety) brain circuits. Firing of dopamine neurons in the dorsomedial hypothalamus and arcuate nucleus inhibits feeding. Feeding elevates extracellular dopamine concentrations in the ventromedial (VM) hypothalamus. A chronic high-fat diet leads to a decrease in dopamine turnover in the hypothalamus. Dopamine receptors 2, 3 and 4 are all "D2-like" receptors and are important in modulating food intake. Both the capacity of dopamine to bind to the D2R and dopamine receptor densities are inversely proportional to the body mass index (BMI).[10] In keeping with this finding, D2R mRNA expression is positively correlated with obesity. In obese individuals (high BMI), there are fewer dopamine receptors to bind with and the dopamine binds less well, which prevents the message to stop eating from getting through. Increasing D2R mRNA activity is an attempt to increase D2R availability as a correction for chronic sedentary behaviour (long-term daily exercise is shown to increase D2R density in rats).[7,8]

Low D2R availability may make the obese less sensitive to stimulation of the DA-regulated reward circuits, which puts them at risk for over-eating. High energy intake obesity (too much food eaten) results in "reward deficiency syndrome" due to a decline in D2Rs. With food intake, dopamine is increased, which leads to an increase in the activity of the dopamine transporter mRNA, particularly in obese individuals. This increase in dopamine transporter production leads to a decrease of dopamine available at the synapse to interact with the dopamine receptor. [8]

In some cases weight gain may be multifactorial. There are numerous orexigenic (appetite stimulating) and anorexigenic (appetite suppressing) peptides and cytokines important in the regulation of food intake. Some of the these peptides and cytokines interact with dopamine and may be affected by some mental illnesses or their treatment.

Leptin is a key anorexigen, produced in fat cells, the placenta, gut and possibly the brain. When body fat is reduced, leptin is also reduced leading to an increase in food intake. When body fat is increased, more leptin is produced, which should lead to satiety. Obesity might be associated with "leptin resistance", similar to insulin resistance. With high body fat, leptin is chronically high and this saturates the active transporter for leptin across the blood-brain barrier and leptin's message to stop eating does not get through. However, if leptin is injected into hypothalamus, feeding is halted. Leptin is highly correlated with body mass index, and increases markedly after puberty, but is mitigated to a large degree by testosterone, so women have 2-3 fold higher leptin levels than men. Physical exercise and fasting decrease leptin, and stimulate feeding. [1]

Leptin and insulin are key signals in the energy storage to the central nervous system. Leptin is involved in brain reward circuits. In the hypothalamus, leptin inhibits neuropeptide Y, a key orexigen, and stimulates several key anorexigens. Noradrenolin neurons are co-localized with neuropeptide Y in the periventrical nucleus of the anterior hypothalamus. In leptin deficiency, noradrenolin in the periventrical nucleus, and perhaps other hypothalamic areas, is increased (leptin normally blocks noradrenolin activity in the hypothalamus), which might be the mechanism responsible for hyperphagia associated with leptin deficiency. If noradrenolin or neuropeptide Y are injected directly into the periventrical nucleus, this results in increased feeding. Increased neuropeptide Y is also associated with increased insulin resistance and a reduced basal metabolic rate (BMR).[1]

Leptin's interaction with dopamine might be related to mesolimbic dopamine projections associated with feeding reward effects. Dopamine agonism in the dorsomedial hypothalamus and arcuate nucleus inhibit feeding and dopamine levels are decreased in the arcuate nucleus of obese mice. Leptin inhibits dopamine secretion from hypothalamic nerve terminals *in vitro.* Firing of dopamine neurons in the dorsomedial hypothalamus and arcuate nucleus inhibits feeding. D2R activation reduces hypothalamic neuropeptide Y mRNA expression, which reduces feeding. A leptin-induced reduction in feeding is antagonized by blocking HI (antihistamine effect), resulting in over-eating and weight gain.

The lateral hypothalamus appears to be a key area in the feeding reward system. Orexins are important orexigens with neurons in the lateral hypothalamus and perifornical area. Orexin promotes feeding and provides an excitatory influence on the dopamine reward system. Orexin might also be involved in the reward system associated with drug dependency. Dopamine inhibits the reward pathway and food intake by acting in the lateral nucleus (LH)/perifornical area. Dopamine receptor activation inhibits feeding, and D2R receptor antagonism blocks the anorexic effects of dopamine in the LH/perifomical area. Anorexic dopamine activity may be due to inhibition of orexin neurons. Antipsychotics that increase the FOS expression of orexin neurons also block dopamine receptors.[1,9]

Dopamine modulates excitatory synaptic transmission in a dose-dependent, reversible manner in the orexin neurons. The direction of the modulation depends on the dopamine receptor type. D1Rs facilitate orexin at low dopamine doses. D2Rs decrease the frequency of spontaneous excitatory neurotransmissions at high dopamine doses. Low to moderate dopamine levels in the LH/perfornical area excites orexin neurons through D1Rs, which excites ventral tegmental area (VTA) neurons, ultimately increasing dopamine release in the nucleus accumbens and prefrontal cortex (a positive feedback loop is created with excitation of the reward pathway and increased appetite). In a parallel fashion, atypical antipsychotics that antagonize 5HT2C receptors (e.g. olanzapine and quetiapine) increase feeding through an excitatory effect on the reward pathway by disinhibiting release of dopamine from the VTN to the nucleus accumbens. A higher dopamine level in the LH/prefornical area activates D2Rs and this inhibits orexin neurons. There is reduced excitation of the VTA neurons and a negative feedback loop is established resulting in an inhibition of the reward pathway (decreased appetite). [1,7,8,9]

A recently published paper investigated the mechanism of action of the oral hypoglycemic metformin and its anorexigenic properties.[11] *In vitro,* metformin blocks phosphorylation of AMP-activated kinase in the hypothalamus, which decreases neuropeptide Y concentrations. This should lead to a decrease in feeding. This effect mirrors that of leptin in the hypothalamus. AMP-activated kinase may mediate some of leptin's peripheral effects and interestingly, metformin, like leptin, has a dual effect, activating AMP-activated kinase in skeletal muscle cells, while inhibiting AMP-activated kinase in the hypothalamus. Metformin has not consistently demonstrated beneficial effects in reversing the weight gain associated with psychotropic medications. [1,11]

Proinflammatory cytokines are essential for the development of anorexia during infectious diseases. These include tumor necrosis factor (TNFα), interleukin 1β (IL-1β) and IL-6. TNFα is synthesized in fat cells, which also produce its receptors. Both TNFα and its receptor are associated with obesity. TNFα may induce insulin resistance and impair glucose tolerance. Clozapine, olanzapine and the antidepressants amitriptyline and mirtazapine are activators of the TNFα system. Drugs that do not always cause weight gain (paroxetine, venlafaxine, haloperidol) are not associated with the activation of the TNFα system. However, no definitive causal mechanism has been found to explain the association between TNFα and obesity. The activation of the TNFα system occurs within the first week of treatment with clozapine and olanzapine and then remains constant. [1]

Effective treatment of many mental illnesses requires long-term patient compliance with drug therapies. Unfortunately, as mentioned above, weight gain often causes patients to terminate treatment prematurely, resulting in a worsening of symptoms. Although the biological mechanisms causing weight gain and associated metabolic syndrome in patients receiving psychotropic medications have not yet been fully elucidated, there is an urgent need for new methods and compositions for preventing or reducing this serious side effect of conventional treatment.

### Summary of the Invention

In accordance with the invention, a method of preventing or reducing one or more of weight gain, type II diabetes, and metabolic syndrome in a patient receiving treatment for a mental illness is described. The method comprises administering to the patient an effective amount of a dopamine agonist in conjunction with an effective amount of an atypical antipsychotic drug. Examples of atypical antipsychotic drugs which may be administered in conjunction with the dopamine agonist include clozapine, olanzapine, quetiapine and risperadone. In one embodiment of the invention, the dopamine agonist is pramipexole. The dopamine agonist may be administered in a low dose, such as less than 1 mg per day of pramipexole.

The invention also relates to compositions comprising an effective amount of a dopamine agonist and an atypical antipsychotic drug in combination, and uses thereof.

### Description

Throughout the following description, specific details are set forth in order to provide a more thorough understanding of the invention. However, the invention may be practiced without these particulars. In other instances, well known elements have not been shown or described in detail to avoid unnecessarily obscuring the invention. Accordingly, the specification and drawings are to be regarded in an illustrative, rather than a restrictive, sense.

This invention relates to methods and compositions for preventing or reducing weight gain and associated metabolic syndrome in patients receiving atypical antipsychotic drugs for treatment of mental illnesses. More particularly, this application relates to the combined administration of one or more atypical antipsychotic drugs and a dopamine agonist. As indicated in the experimental examples below, this combined therapy has been shown to be clinically effective in limiting or reducing weight gain in patients requiring therapy.

One dopamine agonist shown to be effective in accordance with the invention is pramipexole (sold under the trademarks MIRAPEX® and SIFROL®). Pramipexole is a dopamine agonist developed for the treatment of Parkinson's disease and restless leg syndrome. It is metabolized by the kidney and does not interact with the cytochrome p450 (CPY) system. Pramipexole has up to 10 times greater affinity for the D3R when compared to the D2R and 17 times greater affinity when compared to the D4R. It has no affinity for D1 or D5, 5HT, Ach, H1, opioid, α1-adrenergic or β-adrenergic receptors and mild affinity for α2 receptors.[13]

There is some support in the literature for the proposition that administration of pramipexole may influence patient weight. In a randomized controlled trial (RCT) using pramipexole in fibromyalgia, 40% of subjects lost more than 5 pounds over the course of the 14 week study vs. 10% in the placebo group. 21 % gained more than 5 pounds in the pramipexole group vs. 57% in the placebo group.[13] The study used a fixed escalation of dose to 4.5 mg. There were no reported psychotic symptoms or significant sleep disturbances vs. placebo and pramipexole was described as generally well tolerated, although nausea was commonly reported in both groups (79% pramipexole group vs. 71 % in placebo group). Weight loss was not associated with an improvement in fibromyalgia symptoms. The authors noted that the 4.5 mg dose was higher than that typically used to treat restless legs syndrome or Parkinson's disease.[13] Higher concentrations of pramipexole favor post-synaptic dopamine neurotransmission while lower concentrations favor a pre-synaptic effect that inhibits dopamine transmission in the hippocampus. An increase in anxiety, seen in 18 of 38 pramipexole treated subjects and none of the placebo subjects, occurred early in the dose escalation and the authors hypothesized that low dose pramipexole induced anxiety by increasing noradrenolin levels ("adrenergic arousal").

It is possible that low doses of pramipexole have a pre-synaptic effect by lowering levels of norepinephrine and neuropeptide Y, resulting in weight loss. Conversely, higher doses may cause a post-synaptic effect by increasing dopamine agonism, resulting in excitation of the reward pathway and increased feeding and weight gain.

During the premarketing development of pramipexole, patients with earlier or advanced Parkinson's disease were enrolled in clinical trials. Various adverse events were identified, not including significant weight loss or weight gain, and most events were mild or moderate in intensity.[14] One retrospective chart review, published in the Annals of Clinical Psychiatry in September 2000, reported "Pramipexole, used as an adjunct to antidepressants or mood stabilizers, appeared to be effective and safe in the treatment of unipolar and bipolar depression".[15] Accordingly, pramipexole appears to be generally well-tolerated and does not cause any serious side effects which would preclude its use in conjunction with atypical antipsychotics, especially when used at low doses.

Moreover, a randomized controlled trial (RCT) published in Biological Psychiatry in 2004, looking at pramipexole for the treatment of bipolar II patients, found pramipexole to have significant antidepressant effects. In a double-blind, placebo-controlled study, 21 patients with DSM-IV bipolar II disorder, depressive phase on therapeutic levels of lithium or valproate (agents typically referred to as mood stabilizers) were randomly assigned to treatment with pramipexole (n = 10) or placebo (n = 11) for 6 weeks. Primary efficacy was assessed by the Montgomery-Asberg Depression Rating Scale. All subjects except for one in each group completed the study. The analysis of variance for total Montgomery-Asberg Depression Rating Scale scores showed a significant treatment effect. A therapeutic response (>50% decrease in Montgomery-Asberg Depression Rating Scale from baseline) occurred in 60% of patients taking pramipexole and 9% taking placebo (p = .02). One subject on pramipexole and two on placebo developed hypomanic symptoms.[16] Other studies have examined the impact of the addition of pramipexole to an existing treatment regime of conventional antidepressants or mood stabilizers (i.e. not including atypical antipychotics).[21-23]

Considerable data suggest that pramipexole may have D3 preferring effects; these observations are particularly noteworthy, since the D3 receptor has an anatomic distribution that suggests it may play an important role in neuronal circuits that have been implicated in depressive states. In addition to its effects at the D2/D3 receptor, it is now clear that pramipexole also exerts robust neurotrophic effects, many of which may be mediated via upregulation of the antiapoptotic protein Bcl-2.[17,18]

In summary, several different factors support the use of pramipexole to retard weight gain when administered in conjunction with atypical antipsychotics, such as:
1) A fibromyalgia study in which 40% of study subjects lost more than 5 pounds compared to 10% on placebo. As explained above, there were no serious adverse events in this study which had a forced titration to 4.5 mg per day.[13]
2) Pramipexole appears to positively augment the antidepressant effect in mood disordered patients (where atypicals are commonly prescribed) without significant side effects or emerging psychosis.[15,16]
3) Case reports in patients with Parkinson's disease where doses in the range of 3.5-4.5 mg/day caused weight gain which decreased when the dose was lowered. [19] As demonstrated in the examples below, it appears pramipexole may be particularly effective when administered in a low dose. As used in this patent application, a low dose of pramipexole may preferably be in the range of less than 1 mg per day (e.g. 0.125 to 0.5 mg per day). Pre-synaptic and post-synaptic dopamine receptor affinities have been determined for pramipexole, suggesting possible mechanisms of action for weight loss. For example, affinity for pre-synaptic dopamine receptors at low doses and post-synaptic receptors at higher doses may explain why pramipexole prevents weight gain at low doses and possibly promotes weight gain at higher doses.
4) A study showing D2 agonism decreasing hypothalamic orexin, resulting in an inhibitory effect on the reward pathway and decreased feeding. This is opposite to the effect of 5HT2C antagonism which may be involved in weight gain induced by atypical antipsychotics.[1]
5) Clinical observations, described further below, that 0.125 mg pramipexole per day stopped galactorrhea caused by risperidone, suggesting that even small doses are affecting tuberoinfundibular dopamine sufficiently to lower prolactin.
6) Dopamine agonism at the hypothalamic level decreases neuropeptide Y which would result in decreased appetite.[14]

As will be appreciated by a person skilled in the art, dopamine agonists other than pramipexole may also be employed in accordance with the invention. Several other dopamine agonists have been reported to have an effect on food intake and weight. Examples of other dopamine agonists include bromocriptine, cabergoline and pergolide.

Bromocriptine is a dopamine agonist that has been shown to increase insulin sensitivity. After 8 days of bromocriptine, there is a reduction in diurnal glucose and insulin concentrations, lower blood pressure due to the sympatholytic effect of D2R activation, and an increased basal metabolic rate (BMR). Long-term bromocriptine increased glucose tolerance and reduced body fat. Bromocriptine might improve glucose homeostasis by reducing hypothalamic neuropeptide Y or via its sympatholytic properties (increased hypothalamic NA is a marker for obesity and leads to increased feeding). Bromocriptine might also impact glucose metabolism by reducing noradrenolin in the ventromedial hypothalamus. As a result, it has been investigated for its usefulness in treating type II diabetes.[7]

Although the incidence of adverse effects with bromocriptine is quite high (69%), these are generally mild to moderate in degree. Therapy was discontinued in approximately 5% of patients because of adverse effects. These in decreasing order of frequency are: nausea (49%), headache (19%), dizziness (17%), fatigue (7%), lightheadedness (5%), vomiting (5%), abdominal cramps (4%), nasal congestion (3%), constipation (3%), diarrhea (3%) and drowsiness (3%).[12]

Other dopamine agonists which may be used in accordance with the invention include cabergoline, pergolide, ropinirole and quinagolide. Ropinirole has characteristics most similar to pramipexole with similar D2 affinity but less D3 affinity. However, ropinirole does have hepatic metabolism and p450 interactions which complicate its use with other medications. By contrast 90% of pramipexole is excreted unchanged in the urine. A low dose of ropinirole would be 0.25 -1.0 mg. (upper dose ranges 4-6 mg per day).

Quinagolide is a D2 receptor agonist which is much better tolerated than bromocriptine with a half-life of 24 hours allowing once daily dosing which would be favorable if one were creating a combination product with an atypical antipsychotic. One 1991 study when quinagolide was named CV205-502 [20] mentions significant weight loss in 11 of 12 patients treated with this drug for macroprolactinomas. The weight loss could have been related to lowering prolactin levels but there may also be weight loss related to the D2 receptor agonism directly. A low dose for quinagolide would be 25-50 micrograms per day.

The following examples will further illustrate the invention in greater detail although it will be appreciated the invention is not limited to the specific examples.

### Examples

Pramipexole was tested initially in patients who had gained significant weight (20-40 pounds) on olanzapine and who then relapsed after stopping olanzapine treatment. This patient group did not respond to trials of quetiapine or risperidone.

In an attempt to prevent weight gain again (weight had been lost while off olanzapine) these patients were started on 0.125 mg pramipexole per day titrating the dose upwards on a weekly basis until hunger decreased. The highest dose required in 18 patients was 0.25 mg twice per day. Doses of pramipexole between 0.125-0.50 mg per day not only prevented weight gain in these patients started back on olanzapine (for more than 8 weeks) but also have helped patients who have gained weight on olanzapine and quetiapine lose weight. In one patient where fasting blood sugars were higher than normal the pramipexole appears to have been effective in bringing the blood sugars into normal range.

### Example 1

A 31 year-old female was diagnosed with bipolar disorder at age 17. She suffered from panic attacks, obsessive compulsive disorder, and social anxiety. Three years previously, she had gained 60 pounds, raising her weight to 210 pounds, while on carbamazepine and olanzapine (20 mg per day). Two years previously, she had weighed 150 pounds while on lamotrigine alone (400 mg per day).

When the patient was administered pramipexole (0.25 mg twice per day) in combination with olanzapine (20 mg per day), she lost 11 pounds (going from 187 pounds to 176 pounds).

### Example 2

A 34 year-old female suffered from post-traumatic stress disorder with severe depression. She was given quetiapine (300 mg per day), which resulted in her gaining 20 pounds (up to 155 pounds). Therefore, quetiapine was discontinued. Risperidone (2 mg per day) was tried, but she gained 12 pounds more (to 167 pounds) and developed galactorrhea. Therefore, risperidone was discontinued.

The same day that risperadone was discontinued, olanzapine (5 mg per day) and pramipexole (0.125 mg per day) were administered in combination. Galactorrhea stopped within the next week, and weight decreased to 160 pounds. The final dose of pramipexole was 0.25 mg twice per day.

### Example 3

A 34 year-old female was diagnosed with bipolar II disorder, suffering both panic attacks and post-traumatic stress disorder. She failed to respond to augmentation with risperidone and quetiapine and so a trial of olazpapine was started at a dose of 5 mg per day, which resulted in a weight gain from 188 pounds to 202 pounds.

The olanzapine dose was increased to 10 mg per day and was administered in combination with pramipexole at a daily dose of up to 0.75 mg per day. This resulted in a marked decrease in appetite, and her weight was maintained at 202 pounds despite the addition of prednisone at 5 mg per day for joint pain.

### Example 4

A 29 year-old female suffered from major depression and panic attacks. She was given olanzapine (5 mg per day) and pramipexole (0.25 mg twice per day) in combination.

Her initial weight of 168 pounds was maintained. She discontinued pramipexole on her own, and her weight increased to 181 pounds within one week. She restarted pramipexole at 0.25 mg twice daily and her weight decreased to 174 pounds within the next two weeks.

### Example 5

A 30 year-old female suffered from post-traumatic stress disorder, with panic attacks and major depression. Augmentation of Effexor XR (venlafaxine) 225 mg per day with quetiapine (300 mg per day) led to a weight increase to 154 pounds, and was not helping. Quetiapine was therefore discontinued.

The patient was given olanzapine (5 mg per day) and pramipexole (0.125 mg per day) in combination. Weight initially increased to 160 pounds, but a dose increase of pramipexole to 0.25 mg per day resulted in her weight returning to 154 pounds upon reassessment after 6 weeks.

### Example 6

A 46 year-old female suffered from bipolar I disorder, with panic attacks and social and generalized anxiety. She was given quetiapine (300 mg per day) and risperidone (1 mg per day). This resulted in a weight gain from 230 to 247 pounds.

She started pramipexole at 0.25 mg per day after weight gain and her weight decreased to 244 pounds; however, when pramipexole was discontinued, her hunger increased tremendously and her weight increased further to 250 pounds.

### Example 7

A 33 year-old male suffered from major depression with panic attacks. He was given a combination of quetiapine and risperidone for augmentation of antidepressants. His weight increased from 202 to 208 pounds, and there was a worsening of symptoms.

He started olanzapine at 5 mg per day and pramipexole at 0.125 mg per day in combination, and his weight increased to 217 pounds in 2 weeks. The pramipexole dosage was increased to 0.25 mg per day, and the patient's weight decreased to 214 pounds in two weeks, and then to 212 pounds. Cholesterol pre-pramipexole was 6.6 mmol/L and triglycerides were 2.88 mmol/L. After pramipexole, these figures decreased to 5.6 mmol/L and 2.4 mmol/L respectively, with no dietary changes and weight gain.

### Example 8

A 56 year-old male suffered from bipolar II disorder, with post-traumatic stress disorder and panic attacks. He was placed on quetiapine at 375 mg per day for augmentation, and his weight was 259 pounds.

His medication was changed to olanzapine (15 mg per day) and weight was maintained at 259 pounds with pramipexole at 0.25 mg TID (three times per day). With each increase in dose of pramipexole, a marked decrease in his voracious appetite was noticed.

### Example 9

A 28 year-old female suffered from bipolar II disorder, including a depressive mixed state, panic attacks, and obsessive compulsive disorder. She was on lamotrigine (200 mg twice per day). After an increase in quetiapine from 300 mg to 600 mg HS (at bedtime), her weight increased from 150 pounds to 160 pounds.

With pramipexole at 0.25 mg AM & HS (in the morning and at bedtime), weight decreased by 3 pounds and was maintained at 157 pounds.

### Example 10

A 61 year-old male was undergoing treatment for refractory depression and participated in multiple optimized antidepressant trials. He was given Cipralex (40 mg per day) and Wellbutrin (300 mg XL (extended release) per day).

Olanzapine was added at 2.5 mg HS (at bedtime). He gained 2 pounds in 5 days (from 171 to 173 pounds) and noted a marked increase in appetite. Pramipexole at 0.125 mg AM & HS (in the morning and at bedtime) maintained his weight at 173 pounds past two months, despite an increased dose of olanzapine to 5 mg per day over the final month.

### Example 11

A 35 year-old female suffered from bipolar II disorder, with panic attacks and social anxiety. She was on Effexor XR at 300 mg per day for 1 year. She was given quetiapine at 100 mg per day for three months with no weight gain. However, upon increasing quetiapine to 200 mg per day, she gained 10 pounds in two weeks (from 152 pounds to 162 pounds).

On further administration of pramipexole at 0.125 mg once daily to twice per day, she gained no further weight over 3 weeks. After increasing pramipexole to 0.25 mg AM & HS (in the morning and at bedtime) for 3 weeks, she gained 5 pounds. She was asked to decrease dose to 0.125 mg twice per day. This patient was put on prednisone by her gastroenterologist which caused her weight to increase from 167 pounds to 185 pounds. Accordingly, the specific impact of decreasing the pramipexole dose could not be determined.

### Example 12

A 33 year-old female suffered bipolar I disorder. She had suffered from bulimia from age 20 to 22, and suffered social anxiety and body dysmorphic disorder. She developed a rash while on quetiapine in two trials. She was diagnosed with psychotic depression, and was prescribed olanzapine at 5 mg per day, which was increased to 20 mg per day. She was also prescribed lamotrigine and prozac. She gained 37 pounds (from 145 to 182 pounds).

She was started on pramipexole at 0.25 mg twice per day and lost ten pounds (to 172 pounds). After approximately two months on pramipexole, fasting blood sugar (FBS) was 6.6 mmol/L, cholesterol 7.5 mmol/L, and triglycerides 3.6 mmol/L, the same as before starting pramipexole.

### Example 13

A 26 year-old female suffered bipolar NOS (not otherwise specified), with obsessive compulsive disorder and panic attacks. She gained 20 pounds on mirtazapine (45 mg per day), and a further 10 pounds on quetiapine (600 mg HS (at bedtime)) despite her exercise and diet.

She was started on pramipexole up to 0.25 mg twice per day, and lost 5 pounds in three weeks (from 132 to 127 pounds).

### Example 14

A 40 year-old female suffered from post-traumatic stress disorder with severe depression. She was given multiple medications, including Effexor XR, lamotrigine, and benzodiazepine.

To this was added olanzapine (10 mg per day) and pramipexole (0.25 mg twice per day). Her initial weight of 312 pounds was maintained for several months.

### Example 15

A 28 year-old female suffered from bipolar I disorder, with panic attacks and borderline personality disorder. The only agent that had worked previously for mania was olanzapine, which was refused because the patient had previously gained 30 pounds.

The patient agreed to a trial with a combination of olanzapine 2.5 - 5 mg per day and pramipexole 0.125 - 0.25 mg per day, and did not experience weight gain. However, she could not continue with the pramipexole due to cost, and immediately gained weight. As a result, olanzapine was discontinued and the patient was hospitalized.

### Example 16

A 22 year-old female suffered from bipolar I disorder, with panic attacks and borderline personality disorder similar to Example 15. The only agent that had worked previously for mania was olanzapine, which was refused because the patient had previously gained 23 pounds.

The patient agreed to a trial with a combination of olanzapine 2.5 - 5 mg per day and pramipexole 0.125 - 0.25 mg per day, and did not experience weight gain. However, she could not continue with the pramipexole due to cost, and immediately gained weight. As a result, olanzapine was discontinued and the patient was hospitalized.

As will be apparent to those skilled in the art in the light of the foregoing disclosure, may alterations and modifications are possible in the practice of the invention without departing from the spirit or scope thereof. It is therefore intended that the following appended claims and claims hereafter introduced are interpreted to include all such alterations and modifications as are within their true scope.

### ENDNOTES

1. Zimmermann U, Kraus T, Himmerich H, Schuld A, Pollmächer T (2003) Epidemiology, implications and mechanisms underlying drug-induced weight gain in psychiatric patients. J Psychiatr Res 37:193-220.
2. Taylor V, MacQueen G (2006) Associations between bipolar disorder and metabolic syndrome: a review. J Clin Psychiatry 67(7):1034-1041.
3. McElroy SL, Frye MA, Suppes T, Dhavale D, Keck PE, Leverich GS, Altshuler L, Denicoff KD, Nolen WA, Kupka R, Grunze H, Walden J, Post RM (2002) Correlates of overweight and obesity in 644 patients with bipolar disorder. J Clin Psychiatry 63(3):207-213.
4. McElroy SL, Kotwal R, Malhotra S, Nelson EB, Keck PE, Nemeroff CB (2004) Are mood disorders and obesity related? A review for the mental health professional. J Clin Psychiatry 65(5): 634-651.
5. Berkowitz RI, Fabricatore AN (2004) Obesity, Psychiatric status, and psychiatric medications. Psychiatr Clin N Am 28:39-54.
6. Gründer G, Kungel M, Ebrecht M, Göröcs T, Modell S (2006) Aripiprazole: Pharmacodynamics of a dopamine partial agonist for the treatment of schizophrenia. Pharmacopsychiatry 29(Suppl 1):521-525.
7. Kok P, Roelfsema F, Frölich M, van Pelt J, Stokkel MPM, Meinders AE, Piji H (2006) Activation of dopamine D2 receptors simultaneously ameliorates various metabolic features of obese women. Am J Physiol Endocrinol Metab 291:E1038-E1043.
8. Huang XF, Yu Y, Zavitsanou K, Han M, Storlien L (2005) Differential expression of dopamine D2 and D4 receptor and tyrosine hydroxylase mRNA in mice prone, or resistant, to chronic high-fat diet-induced obesity. Molecular Brain Research 135(1-2):150-161.
9. Alberto CO, Trask RB, Quinlan ME, Hirasawa M (2006) Bidirectional dopaminergic modulation of excitatory synaptic transmission in orexin neurons. J Neurosci 26(39):10043-10050.
10. Wang GJ, Volkow ND, Fowler JS (2002) The role of dopamine in motivation for food in humans: implications for obesity. Expert Opin Ther Targets 6(5): 601-9.
11. Chau-Van C, Gamba M, Salvi R, Gaillard RC, Pralong FP (2007) Metformin inhibits adenosine 5'-monophosphate-activated kinase activation and prevents increases in neuropeptide Y expression in cultured hypothalamic neurons. Endocrinology 148(2):507-11. Epub 2006 Nov 9.
12. Compendium of Pharmaceuticals and Specialties (CPS) 2007. Canadian Pharmacists Association. Ottawa, Ontario, Canada
13. Holman AJ, Myers RR (2005) A randomized, double-blind, placebo-controlled trial of pramipexole, a dopamine agonist, in patients with fibromyalgia receiving concomitant medications. Arthritis & Rheumatism 52(8):2495-2505.
14. Jerzy Maj, Zofia Rogoz, Grazyna Skuza, Krzysztof Kolodziejczyk (1997) The behavioural effects of pramipexole, a novel dopamine receptor agonist. European Journal of Pharmacology 324:31-37
15. Sporn J, Ghaemi SN, Samburm R, et al (2000). Pramipexole augmentation in the treatment of unipolar and bipolar depression; A retrospective chart review. Ann Clin Psychiatry 12:137-140
16. Zarate CA, Payne JL, Singh J, Quiroz JA, Luckenbaugh DA, Denicoff KD, Charney DS, Manji HK (2004) Pramipexole for bipolar II depression: A placebo-controlled proof of concept study. Biol Psychiatry 56:54-60.
17. Carvey PM, McGuire SO, Ling ZD (2001) Neuroprotective effects of D3 dopamine receptor agonists. Parkinsonism and Related Disorders 7:213-223.
18. Gupta S, Vincent JL, Frank B (2006) Pramipexole: augmentation in the treatment of depressive symptoms. CNS Spectr 11(3):172-175.
19. Melissa J. Nirenberg, MD, Cheryl Waters (2006) Compulsive Eating and Weight Gain Related to Dopamine Agonist Use. Movement Disorders Vol. 21, No. 4, 2006, pp. 524-529.
20. Barnett PS, Palazidou E, Miell JP, Coskeran PB, Butler J, Dawson JM, Maccabe J, McGregor AM (1991) Endocrine function, psychiatric and clinical consequences in patients with macroprolactinomas after long-term treatment with the new non-ergot dopamine agonist CV205-502. Q J Med. 1991 Nov 81(295):891-906.
21. Perugi G, Toni C, Ruffolo G, Frare F, Akiskal H (2001) Adjunctive dopamine agonists in treatment-resistant bipolar II depression: an open case series. Pharmacopsychiatry 34: 137-141.
22. Lattanzi L, Dell'Osso L, Cassano P, et al. (2002) Pramipexole in treatment-resistent depression: a 16-week naturalistic study. Bipolar Disorders 4:307-314.
23. Goldberg JF, Burdick KE, Endick CJ (2004) Preliminary randomized, double-blind, placebo-controlled trial of pramipexole added to mood stabilizers for treatment-resistant bipolar depression. Am J Psychiatry 161(3):564-566.

### EMBODIMENTS:

1. A method of preventing or reducing one or more of weight gain, type II diabetes, and metabolic syndrome in a patient receiving treatment for a mental illness, the method comprising administering to the patient an effective amount of a dopamine agonist in conjunction with an effective amount of an atypical antipsychotic drug.
2. A method as defined in embodiment 1 wherein the dopamine agonist is selected from the group consisting of pramipexole, bromocriptine, cabergoline, pergolide, ropinirole and quinagolide.
3. A method as defined in embodiment 2 wherein the dopamine agonist is pramipexole.
4. A method as defined in embodiment 2 wherein the dopamine agonist is quinagolide.
5. A method as defined in any one of embodiments 1-4 wherein the atypical antipsychotic drug is selected from the group consisting of clozapine, olanzapine, quetiapine, and risperadone.
6. A method as defined in embodiment 5 wherein more than one atypical antipsychotic is administered.
7. A method as defined in embodiment 1 wherein the atypical antipsychotic drug antagonizes the 5HT2 receptor to a greater extent than it antagonizes the DA2 receptor.
8. A method as defined in embodiment 1 wherein a low dose of the dopamine agonist is administered.
9. A method as defined in embodiment 3 wherein the dose of pramipexole is less than one milligram per day.
10. A method as defined in embodiment 9 wherein the dose of pramipexole is within the range of 0.125 to 0.5 mg per day.
11. A method as defined in embodiment 4 wherein the dose of quinagolide is in the range of 25 to 50 micrograms per day.
12. A method as defined in embodiment 1 wherein the dopamine agonist is ropinirole.
13. A method as defined in embodiment 12 wherein the dose of ropinirole is in the range of 0.25 to 1.0 milligrams per day.
14. A method as defined in any one of embodiments 1-13 wherein the mental illness is selected from the group consisting of bipolar I disorder, bipolar II disorder, obsessive-compulsive disorder, panic attacks, social anxiety, post traumatic stress disorder, and depression.
15. A method of preventing or reducing weight gain in a patient receiving an atypical antipsychotic drug for treatment of a mental disorder, the method comprising administering an effective amount of a compound having a dopamine agonist effect in conjunction with the atypical antipsychotic drug.
16. A composition comprising an effective amount of a dopamine agonist and an effective amount of an atypical antipsychotic drug.
17. A composition as defined in embodiment 16 wherein the dopamine agonist is selected from the group consisting of pramipexole, bromocriptine, cabergoline, pergolide, ropinirole and quinagolide.
18. A composition as defined in embodiment 17 wherein the dopamine agonist is pramipexole.
19. A composition as defined in embodiment 17 wherein the dopamine agonist is quinagolide.
20. A composition according to any one of aspects 16-19 wherein the atypical antipsychotic drug is selected from the group consisting of clozapine, olanzapine, quetiapine and risperadone.
21. A composition as defined in any one of embodiments 16-20 further comprising a pharmaceutically effective carrier.
22. A composition as defined in any one of embodiments 16-18, 20 or 21,wherein the dopamine agonist is pramipexole in a unit dosage form less than 1 mg.
23. The use of a dopamine agonist in combination with an atypical antipsychotic drug for preventing or reducing weight gain associated with administration of the antipsychotic drug.
24. A use as defined in embodiment 23 wherein the dopamine agonist is pramipexole.
25. A use as defined in embodiment 23 wherein the dopamine agonist is quinagolide.
26. A use as defined in embodiment 23 wherein the atypical antipsychotic drug is selected from the group consisting of bromocriptine, clozapine, olanzapine, quetiapine and risperadone.

## Claims

1. A composition comprising an effective amount of a dopamine agonist and an effective amount of an atypical antipsychotic drug.

2. The composition according to claim 1 wherein the dopamine agonist is selected from the group consisting of pramipexole, bromocriptine, cabergoline, pergolide, ropinirole and quinagolide, and preferably is pramipexole and quinagolide.

3. The composition according to claims 1 or 2 wherein the atypical antipsychotic drug is selected from the group consisting of clozapine, olanzapine, quetiapine and risperadone.

4. The composition according to any one of claims 1-3 further comprising a pharmaceutically effective carrier.

5. The composition according to any one of claims 1-4, wherein the dopamine agonist is pramipexole in a unit dosage form less than 1 mg.

6. The composition according to any of claims 1-5 for use in the prevention or reduction of one or more of weight gain, type II diabetes, and metabolic syndrome in a patient receiving treatment for a mental illness.

7. The composition of claim 6, wherein more than one atypical antipsychotic drug is administered.

8. The composition of claim 6, wherein the atypical antipsychotic drug antagonizes the 5HT2 receptor to a greater extent than it antagonizes the DA2 receptor.

9. The composition of claim 6, wherein a low dose of the dopamine agonist is administered.

10. The composition of claim 6, wherein the dose of pramipexole is within the range of 0.125 to 0.5 mg per day, or
wherein the dose of quinagolide is in the range of 25 to 50 micrograms per day, or wherein the dopamine agonist is ropinirole being in the range of 0.25 to 1.0 milligrams per day.

11. The composition of claim 6, wherein the mental illness is selected from the group consisting of bipolar I disorder, bipolar II disorder, obsessive-compulsive disorder, panic attacks, social anxiety, post traumatic stress disorder, and depression.

12. The composition of claim 6 for use in the prevention or reduction of weight gain in a patient receiving an atypical antipsychotic drug for treatment of a mental disorder.
